# EUROPEAN PATENT APPLICATION

(11) **EP 3 680 339 A1**
(43) Date of publication of application: **15.07.2020**
(21) Application number: 19151402.5
(22) Date of filing: 11.01.2019
(51) Int. Cl.: C12P 13/00

(54) **FERMENTATIVE PRODUCTION OF PYRAZINES USING MICROORGANISMS OF THE GENUS PASTEURELLACEAE**

(71) Applicant: BASF SE, 67056 Ludwigshafen am Rhein (DE)
(72) Inventor: Pelzer, Ralf, 68623 Lampertheim (DE); Zorn, Holger, 35392 Gießen (DE); Birk, Florian, 35390 Gießen (DE); Fraatz, Marco Alexander, 35390 Gießen (DE)
(74) Representative: Altmann Stößel Dick Patentanwälte PartG mbB

(57) **Abstract**

The present invention relates to a process for producing pyrazines of the general formula (I) 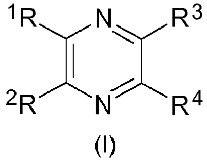 in which R¹, R², R³ and R⁴ independently from each other represent hydrogen, a C₁-C₁₀ alkyl group, a C₁-C₁₀ alkoxy group or a C₁-C₁₀ thioalkoxy group, wherein the process comprises the step of
i) cultivating microorganisms of the family *Pasteurellaceae* in a culture medium comprising at least one assimilable carbon source to allow the microorganisms to produce pyrazines of the general formula (I);
ii) recovering the pyrazines of the general formula (I) from the fermentation broth obtained in process step ii).

The present invention also relates to the use of microorganisms of the family *Pasteurellaceae* for the fermentative production of such pyrazines.

## Description

The present invention relates to a process for producing pyrazines of the general formula (I) 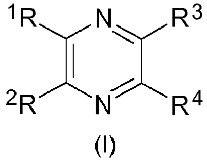 in which R¹, R², R³ and R⁴ independently from each other represent hydrogen, a C₁-C₁₀ alkyl group, a C₁-C₁₀ alkoxy group or a C₁-C₁₀ thioalkoxy group and to the use of microorganisms of the family *Pasteurellaceae* for the fermentative production of such pyrazines.

Alkylpyrazines are heterocyclic, nitrogen-containing compounds found in a wide variety of foods, which are responsible for different flavors, mainly nutty and roasty, and are thus used in the food industry as additives for flavoring. 2,5-dimethyl pyrazine and tetramethyl pyrazine are, for example, the main pyrazines detected in many cocoa bean- or soybean-based fermented foods, where they are recognized to be important contributors to their flavor.

There have also been attemps to use microorganisms, including bacteria and fungi, for the fermentative production of pyrazines using carbohydrates as carbon sources. Dickschat et al. (Eur. J. Org. Chem. 2010, 2687-2695), for example, describe the biosynthesis of pyrazines in *Corynebacterium glutamicum* cultured on glucose as the main carbon source. However, the preceding attemps of using microorganisms in liquid media resulted in low concentrations of pyrazines in the fermentation broths, hampering an industrial application of this approach so far.

It was therefore an object of the present invention to overcome the disadvantages of the prior art.

In particular, it was an object of the present invention to provide a process for producing pyrazines by fermentation, particularly pyrazines selected from the group consisting of 2,3,5-trimethyl pyrazine, 2,3,5-trimethyl-6-ethyl pyrazine, 2,3,5-trimethyl-6-propyl pyrazine and 2,3,5,6-tetramethyl pyrazine, which allows the production of these compounds in higher concentrations, compared to the processes known in the prior art.

Furthermore, it was an object of the present invention to provide a process for producing pyrazines by fermentation which allows the production of these compounds using inexpensive and easily accessible carbon sources such as glucose or sucrose.

A contribution to achieving the abovementioned aims is provided by a process for producing pyrazines of the general formula (I) 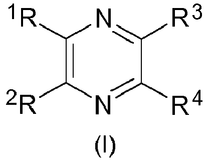 in which R¹, R², R³ and R⁴ independently from each other represent hydrogen, a C₁-C₁₀ alkyl group, preferably a C₁-C₅ alkyl group and more preferably a methyl group, an ethyl group or a propyl group, a C₁-C₁₀ alkoxy group, preferably a C₁-C₅ alkoxy group and more preferably a methoxy group or an ethoxy group, or a C₁-C₁₀ thioalkoxy group, preferably a C₁-C₅ thioalkoxy group and more preferably a thiomethoxy group or a thioethoxy group, wherein the process comprises the step of
i) cultivating microorganisms of the family *Pasteurellaceae* in a culture medium comprising at least one assimilable carbon source to allow the microorganisms to produce pyrazines of the general formula (I);
ii) recovering the pyrazines of the general formula (I) from the fermentation broth obtained in process step i).

In process step i) of the process according to the present invention microorganisms of the family *Pasteurellaceae* are cultivated in a culture medium comprising at least one assimilable carbon source to allow the microorganisms to produce pyrazines of the general formula (I), thereby obtaining a fermentation broth comprising these pyrazines.

*"Pasteurellaceae"* comprise a large family of Gram-negative Proteobacteria with members ranging from bacteria such as *Haemophilus influenzae* to commensals of the animal and human mucosa. Most members live as commensals on mucosal surfaces of birds and mammals, especially in the upper respiratory tract. *Pasteurellaceae* are typically rod-shaped, and are a notable group of facultative anaerobes. They can be distinguished from the related *Enterobacteriaceae* by the presence of oxidase, and from most other similar bacteria by the absence of flagella. Bacteria in the family *Pasteurellaceae* have been classified into a number of genera based on metabolic properties and there sequences of the 16S RNA and 23S RNA. Many of the *Pasteurellaceae* contain pyruvate-formate-lyase genes and are capable of anaerobically fermenting carbon sources to organic acids.

According to a preferred embodiment of the process according to the present invention the microorganism used in process step i) belongs to the genus *Basfia,* more preferably to the species *Basfia succiniciproducens.*

Most preferably, the microorganism that is used in process step i) is *Basfia succiniciproducens*strain DD1 deposited under the Budapest Treaty with DSMZ (Deutsche Sammlung von Mikroorganismen und Zellkulturen, GmbH), Germany, having the deposit number DSM 18541. This strain has been originally isolated from the rumen of a cow of German origin. *Pasteurella* bacteria can be isolated from the gastro-intestinal tract of animals and, preferably, mammals. Further strains of the genus *Basfia* that can be used in process step i) of the process according to the present invention are the Basfia-strain that has been deposited under the deposit number DSM 22022.

In this context it is particularly preferred that the microorganism that is used in process step i) has a 16S rDNA of **SEQ ID NO: 1** or a sequence, which shows a sequence homology of at least 96 %, at least 97 %, at least 98 %, at least 99 % or at least 99.9% with **SEQ ID NO: 1.** It is also preferred that the microorganism that is used in process step i) has a 23S rDNA of **SEQ ID NO: 2** or a sequence, which shows a sequence homology of at least 96 %, at least 97 %, at least 98 %, at least 99 % or at least 99.9 % with **SEQ ID NO: 2.**

The identity in percentage values referred to in connection with the various polypeptides or polynucleotides to be used for the modified microorganism according to the present invention is, preferably, calculated as identity of the residues over the complete length of the aligned sequences, such as, for example, the identity calculated (for rather similar sequences) with the aid of the program needle from the bioinformatics software package EMBOSS (Version 5.0.0, http://emboss.source-forge.net/what/) with the default parameters which are, i.e. gap open (penalty to open a gap): 10.0, gap extend (penalty to extend a gap): 0.5, and data file (scoring matrix file included in package): EDNAFUL.

It should be noted that the microorganism that is used in process step i) may, according to the preferred embodiment of the process, no necessarily be the above described strain *Basfia succiniciproducens-strain* DD1, but can also be a variant of this strain. In this context the expression "a *variant of a strain"* comprises every strain having the same or essentially the same characteristics as the wildtype-strain. In this context it is particularly preferred that the 16 S rDNA of the variant has an identity of at least 90 %, preferably at least 95 %, more preferably at least 99 %, more preferably at least 99.5 %, more preferably at least 99.6 %, more preferably at least 99.7 %, more preferably at least 99.8% and most preferably at least 99.9 % with the wildtype from which the variant has been derived. It is also particularly preferred that the 23 S rDNA of the variant has an identity of at least 90 %, preferably at least 95 %, more preferably at least 99 %, more preferably at least 99.5 %, more preferably at least 99.6 %, more preferably at least 99.7 %, more preferably at least 99.8 % and most preferably at least 99.9 % with the wildtype from which the variant has been derived. A variant of a strain in the sense of this definition can, for example, be obtained by treating the wildtype-strain with a mutagenizing chemical agent, X-rays, or UV light.

The microorganisms used in process step i) may also be genetically altered, modified or engineered (e.g., genetically engineered) such that they exhibit an altered, modified or different genotype and/or phenotype (e. g., when the genetic modification affects coding nucleic acid sequences of the microorganism) as compared to the naturally-occurring wildtype microorganism from which they have been derived. In this context it is particularly preferred that the microorganism used in process step i) is a genetically modified microorganism in which at least one of the genes selected from the group consisting of *IdhA, pflA, pflD, wcaJ, pykA, fruA, ptsA, ptsH, udhA* and *rbsK* has been genetically modified.

The assimilable carbon source is preferably selected from sucrose, maltose, D-fructose, D-glucose, D-xylose, L-arabinose, D-galactose, D-mannose, glycerol and mixtures thereof or compositions containing at least one of said compounds, or is selected from decomposition products of starch, cellulose, hemicellulose and/or lignocellulose. Preferably, the assimilable carbon source comprises D-glucose, sucrose, glycerol or a mixture of at least two of these compounds, wherein mixtures of glycerol and D-glucose, glycerol and sucrose, glycerol and D-xylose and D-glucose and D-fructose are particularly preferred. The initial concentration of the assimilable carbon source is, preferably, adjusted to a value in a range of 5 to 100 g/l, preferably 5 to 75 g/l and more preferably 5 to 50 g/l and may be maintained in said range during cultivation.

The microorganisms are preferably incubated in the culture medium at a temperature in the range of about 10 to 60°C or 20 to 50°C or 30 to 45°C at a pH of 5.0 to 9.0 or 5.5 to 8.0 or 6.0 to 7.0.

Preferably, the pyrazines of the general formula (I) are produced under anaerobic conditions. Anaerobic conditions may be established by means of conventional techniques, as for example by degassing the constituents of the reaction medium and maintaining anaerobic conditions by introducing carbon dioxide or nitrogen or mixtures thereof and optionally hydrogen at a flow rate of, for example, 0.1 to 1 or 0.2 to 0.5 vvm. Aerobic conditions may be established by means of conventional techniques, as for example by introducing air or oxygen at a flow rate of, for example, 0.1 to 1 or 0.2 to 0.5 vvm. If appropriate, a slight over pressure of 0.1 to 1.5 bar may be applied in the process.

The initial concentration of the assimilable carbon source is preferably adjusted to a value in a range of 5 to 100 g/l, preferably 5 to 75 g/l and more preferably 5 to 50 g/l and may be maintained in said range during cultivation. The pH of the reaction medium may be controlled by addition of suitable bases as for example, gaseous ammonia, NH₄HCO₃, (NH₄)₂CO₃, NaOH, Na₂CO₃, NaHCO₃, KOH, K₂CO₃, KHCO₃, Mg(OH)₂, MgCO₃, Mg(HCO₃)₂, Ca(OH)₂, CaCO₃, Ca(HCO₃)₂, CaO, CH₆N₂O₂, C₂H₇N and/or mixtures thereof. These alkaline neutralization agents are especially required if organic compounds such as carboxylic acids or dicarboxylic acids are formed in the course of the fermentation process. In the case of succinic acid as the organic compound, Mg(OH)₂ and MgCO₃ area particular preferred bases.

The fermentation step i) according to the present invention can, for example, be performed in stirred fermenters, bubble columns and loop reactors. A comprehensive overview of the possible method types including stirrer types and geometric designs can be found in Chmiel: "Bioprozesstechnik: Einführung in die Bioverfahrenstechnik", Volume 1. In the process according to the present invention, typical variants available are the following variants known to those skilled in the art or explained, for example, in Chmiel, Hammes and Bailey: "Biochemical Engineering*",* such as batch, fed-batch, repeated fed-batch or else continuous fermentation with and without recycling of the biomass. Depending on the production strain, sparging with air, oxygen, carbon dioxide, hydrogen, nitrogen or appropriate gas mixtures may be effected in order to achieve good yield (YP/S).

In process step ii) the pyrazines of the general formula (I) are recovered from the fermentation broth obtained in process step i).

Usually, the recovery process comprises the step of separating the microorganisms from the fermentation broth as the so called "biomass". Processes for removing the biomass are known to those skilled in the art, and comprise filtration, sedimentation, flotation or combinations thereof. Consequently, the biomass can be removed, for example, with centrifuges, separators, decanters, filters or in a flotation apparatus. For maximum recovery of the product of value, washing of the biomass is often advisable, for example in the form of a diafiltration. The selection of the method is dependent upon the biomass content in the fermentation broth and the properties of the biomass, and also the interaction of the biomass with the organic compound (e. the product of value). In one embodiment, the fermentation broth can be sterilized or pasteurized. In a further embodiment, the fermentation broth is concentrated. Depending on the requirement, this concentration can be done batch wise or continuously. The pressure and temperature range should be selected such that firstly no product damage occurs, and secondly minimal use of apparatus and energy is necessary. The skillful selection of pressure and temperature levels for a multistage evaporation in particular enables saving of energy.

According to a preferred embodiment of the process according to the present invention recovering the pyrazines of the general formula (I) from the fermentation broth in process step ii) comprises, optionally in addition to the above described step of separating the microorganisms from the fermentation broth, at least one treatment step selected from the group consisting of evaporation, filtration, sedimentation, crystallization, steam distillation, solvent extraction, dialysis, treatment with activated carbon, reverse osmosis, chromatography and a combination of at least two of these treatment steps.

In a first embodiment of the process according to the present invention recovering the pyrazines of the general formula (I) from the fermentation broth in process step ii) comprises the steps of
iia1) optionally evaporating the volatile components from the fermentation broth under reduced pressure at a temperature in the range from 50 to 90°C and collecting the evaporated components;
iib1) extracting the fermentation broth obtained in process step ii) or the evaporated components obtained in process step iia) with a solvent to obtained a solvent extract and evaporating the solvent from the solvent extract to obtain a residue comprising pyrazines of the general formula (I);
iic1) separating the pyrazines of the general formula (I) contained in said residue by means of chromatography.

In this context it may also be advantageous if the microorganisms are at least partially removed from the fermentation broth before process step iia1) is performed.

In a second embodiment of the process according to the present invention recovering the pyrazines of the general formula (I) from the fermentation broth in process step ii) comprises the steps of
iia2) separating the microorganisms from the fermentation broth;
iib2) treatment of the fermentation broth obtained in process step iia2) with activated carbon and separating the activated carbon from the fermentation broth;
iic2) treatment of the fermentation broth obtained in process step iib2) with reverse osmosis;
iid2) evaporation of at least a part of the water contained in the permeate obtained in process step iic2) to obtain a concentrated permeate;
iie2) crystallization of pyrazines of the general formula (I) from the concentrated permeate obtained in process step iid2).

In the process according to the present invention it is furthermore preferred that the pyrazines of the general formula (I) are selected from the group consisting of 2,3,5,6-tetramethyl pyrazine, 2,3,5-trimethyl-6-ethyl pyrazine, 2,3,5-trimethyl-6-propylpyrazine, 2,3,5-trimethyl-6-butyl pyrazine, 2,3,5-trimethyl pyrazine, 5-ethyl-2,3-dimethyl pyrazine, 2,5-dimethyl-3-ethyl pyrazine, 2,5-dimethyl-3-propyl pyrazine, 3,5-dimethyl-2-ethyl pyrazine, 2-ethyl-6-methyl pyrazine, 2-methyl-6-ethyl pyrazine, 2,3-dimethyl pyrazine, 2,5-dimethyl pyrazine, 2,6-dimethyl pyrazine, 2,3-diethyl pyrazine, 2,5-diethyl pyrazine, 2,6-diethyl pyrazine, and mixtures of at least two thereof, wherein 2,3,5-trimethyl pyrazine, 2,3,5-trimethyl-6-ethyl pyrazine, 2,3,5-trimethyl-6-propyl pyrazine, 2,3,5,6-tetramethyl pyrazine and mixtures of at least two thereof are particularly preferred and 2,3,5,6-tetramethyl pyrazine is most preferred as a pyrzine of the general formula (I).

A contribution to solving the problems mentioned at the outset is furthermore provided by the use of microorganisms of the family *Pasteurellaceae* for the fermentative production of pyrazines of the general formula (I) 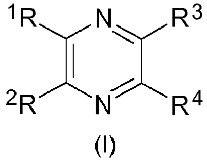 in which R¹, R², R³ and R⁴ independently from each other represent hydrogen, a C₁-C₁₀ alkyl group, preferably a C₁-C₅ alkyl group and more preferably a methyl group, an ethyl group or a propyl group, a C₁-C₁₀ alkoxy group, preferably a C₁-C₅ alkoxy group and more preferably a methoxy group or an ethoxy group, or a C₁-C₁₀ thioalkoxy group, preferably a C₁-C₅ thioalkoxy group and more preferably a thiomethoxy group or a thioethoxy group.

Preferred microorganisms and preferred pyrazines of the general formula (I) are those microorganisms and pyrazines that have been described as preferred embodiments of the process according to the present invention.

The invention is now explained in more detail with the aid of non-limiting examples.

### EXAMPLES

### a) Medium preparation

The composition and preparation of the cultivation medium used for seed culture is as described in the following table 1 and 2. For the main culture in the fermenter a medium is used as described in table 3.

**Table 1: Medium composition for cultivation of the seed culture in the first step**

| Compound | Concentration [g/L] |
|---|---|
| Yeast extract (Bio Springer) | 12.5 |
| (NH₄)₂SO₄ | 5 |
| succinic acid | 2.5 |
| Na₂CO₃ | 2 |
| KH₂PO₄ | 1 |
| MgCO₃ | 50 |
| glucose | 52 |

**Table 2: Medium composition for cultivation of the seed culture in the second step**

| Compound | Concentration [g/L] |
|---|---|
| (NH₄)₂SO₄ | 5 |
| Na₂CO₃ | 2 |
| KH₂PO₄ | 1 |
| MgCO₃ | 50 |
| glucose | 50 |
| vitamins | table 6 |
| trace metals | table 7 |
| osmolytes | table 8 |

**Table 3: Medium composition for the main culture in fermenters**

| Compound | Concentration [g/L] |
|---|---|
| (NH₄)₂SO₄ | 5 |
| Na₂CO₃ | 2 |
| KH₂PO₄ | 1 |
| vitamins | table 4 |
| trace metals | table 5 |
| osmolytes | table 6 |
| polypropylene glycol (antifoam) | 0.05 |
| glucose | 10 |
| glycerol | 50 |

**Table 4: Vitamins used in the second step seed culture and in the main culture**

| Compound | Concentration in the medium [mg/L] |
|---|---|
| thiamine (B1) | 30 |
| riboflavin (B2) | 6 |
| nicotinic acid (B3) | 30 |
| pantothenic acid (B5) | 100 |
| pyridoxin (B6) | 10 |
| biotin (B7) | 5 |
| cyanocobalmin (B12) | 5 |

**Table 5: Trace elements added in the defined lean medium**

| Compound | Concentration in the medium [mg/L] |
|---|---|
| FeSO₄ | 2.04 |
| CaCl₂ | 12.46 |
| MnCl₂ · 4 H₂O | 1.2 |
| CuCl₂ · 2 H₂O | 0.188 |
| ZnSO₄ | 1.851 |
| Na₂Mo₄ · 2 H₂O | 0.038 |
| CoCl₂ · 6 H₂O | 0.101 |
| NiCl₂ · 6 H₂O | 0.032 |
| NaSeO₃ | 0.01 |

**Table 6: Osmolytes used in the second step seed culture and in the main culture**

| Compound | Concentration in the medium (mM) |
|---|---|
| Prolin | 1 |
| Betain | 1 |
| Carnitin | 1 |
| Glutamate | 1 |
| (2-carboxyethyl)dimethylsulfonium chloride | 1 |

### b) Cultivations

For the culture experiments, *Basfia succiniciproducens* DD1 cells have been used.

The main culture is inoculated from a seed train consisting of two seed cultures. For the first seed culture 2.5% of cryo stocks was inoculated in a 100 ml-serum bottle with gas tight butyl rubber stopper containing 50 ml of the liquid medium described in Table 3 with a CO₂ atmosphere with 0.8 bar overpressure. The starting pH of the medium is in the range of 7.5 to 8 due to the MgCO₃ used as base and due to the CO₂ gassing. The incubation was overnight at 37°C under anaerobic conditions. This first seed culture was used to inoculate the second one to OD600 = 0.75 in a 100 ml-serum bottle with gas tight butyl rubber stopper containing 50 ml of the liquid medium described in table 4. CO₂ atmosphere with 0.8 bar overpressure was also applied and the starting pH is in the range of 7.5 to 8. The bottles were incubated at 37°C, and 160 rpm (shaking diameter: 2.5 cm).

For growing the main culture bacteria, the second seed culture (incubated overnight at 37°C under anaerobic conditions) was used as inoculum in a concentration of 10%. The medium used is described in Table 3.

The fermentation was done in 5L fermenters containing an initial volume of 2L. Glycerol and glucose were used as carbon sources and were provided by both batch and feed. The base utilized was magnesium hydroxide 25% or a higher concentration (50% or 62%) and the pH was kept constant at 6.5. CO₂ was applied in the fermenter at flow of 0.1 vvm and the steering rate is 500 rpm. The feeding rates were 0.67 g/(Lh) of glucose and between 2.5 - 5 g/(Lh) for glycerol. After 36h of fermentation a sample of the fermentation broth (5 ml) has been taken in order to quantify the amount of pyrazine that has been formed.

### c) Compound quantitation

The quantitation of compounds present in the sample of the fermentation broth was performed by stir bar sorptive extraction (SBSE) TDU-GC-MS using the following conditions:

**Table 7: GC parameters used for the quantitation by SBSE**

| | |
|---|---|
| gas chromatograph | Agilent Technologies (Waldbronn, Germany) 7890B |
| column | Agilent VF-WAXms |
| | 30 m x 0.25 mm ID, 0.25 µm film thickness |
| carrier gas | helium (5.0), 1.2 mL min⁻¹ (constant) |
| autosample | GERSTEL (Mülheim an der Ruhr, Germany) MPS robotic |
| thermal desorption unit | GERSTEL Thermal desorption unit |
| | splitless |
| | 30 °C (0.5 min), 10 °C s⁻¹ to 240 °C (3 min) |
| | transferline: 250 °C (fixed) |
| | septum purge: 3 mL min⁻¹ |
| injection system | cooled injection system GERSTEL KAS 4 |
| | Tenax liner split ratio: 10:1 |
| | -70 °C, 12 °C s⁻¹ to 250 °C (3 min) |
| temperature program | 40 °C (3 min), 5 °C min⁻¹ to 250 °C (7 min) |
| detector | mass spectrometer (MS): Agilent MSD 5977B Quadrupole |
| temperature MS | transfer line: 250 °C, ion source 230 °C, quadrupoles: 150 °C |
| detection mode MS | scan: *m*/*z* 33 - 300 |
| ionization energy | 70 eV |
| software | MassHunter GC/MS Acquisition B.05.01 |
| | MassHunter Qualitative Analysis B.04.00 |
| database | NIST2011 MS |

5 mL fermentation broth that have been taken as a sample were extracted for 90 min at room temperature by a polydimethyl siloxane (PDMS) twister. The compounds of interest were quantified by standard addition. The results are given in tab. 8.

**Table 8: quantitation of the compounds of interest by SBSE**

| compound | retention index* | | [mg/L] |
|---|---|---|---|
| | VF-WAXms | DB-5 | |
| 2,3-butandione | 973 | <700 | 5.21 |
| 2,3,5-trimethyl pyrazine | 1400 | 1002 | 0.55 |
| 5-ethyl-2,3-dimethyl pyrazine | 1458 | 1088 | n.d. |
| 2,3,5,6-tetramethyl pyrazine | 1470 | 1085 | 182.06 |
| 2,3,5-trimethyl-6-ethyl pyrazine | 1509 | 1157 | 2.75 |
| benzaldehyde | 1521 | 964 | 0.01 |
| 2,3,5-trimethyl-6-propyl pyrazine | 1577 | 1237 | 22.00 |

| | | | |
|---|---|---|---|
| * The retention indices were calculated by comparison of retention time of the standard compounds compared to n-alkanes (C₇-C₃₀). | | | |

### SEQUENCES

**SEQ ID NO: 1** (nucleotide sequence of 16 S rDNA of strain DD1) 
tttgatcctggctcagattgaacgctggcggcaggcttaacacatgcaagtcgaacggtagcgggaggaaagcttgctttctttgccga
cgagtggcggacgggtgagtaatgcttggggatctggcttatggagggggataacgacgggaaactgtcgctaataccgcgtaatat
cttcggattaaagggtgggactttcgggccacccgccataagatgagcccaagtgggattaggtagttggtggggtaaaggcctacc
aagccgacgatctctagctggtctgagaggatgaccagccacactggaactgagacacggtccagactcctacgggaggcagca
gtggggaatattgcacaatgggggaaccctgatgcagccatgccgcgtgaatgaagaaggccttcgggttgtaaagttctttcggtg
acgaggaaggtgtttgttttaataggacaagcaattgacgttaatcacagaagaagcaccggctaactccgtgccagcagccgcggt
aatacggagggtgcgagcgttaatcggaataactgggcgtaaagggcatgcaggcggacttttaagtgagatgtgaaagccccgg
gcttaacctgggaattgcatttcagactgggagtctagagtactttagggaggggtagaattccacgtgtagcggtgaaatgcgtagag
atgtggaggaataccgaaggcgaaggcagccccttgggaagatactgacgctcatatgcgaaagcgtggggagcaaacaggatt
agataccctggtagtccacgcggtaaacgctgtcgatttggggattgggctttaggcctggtgctcgtagctaacgtgataaatcgacc
gcctggggagtacggccgcaaggttaaaactcaaatgaattgacgggggcccgcacaagcggtggagcatgtggtttaattcgatg
caacgcgaagaaccttacctactcttgacatccagagaatcctgtagagatacgggagtgccttcgggagctctgagacaggtgctg
catggctgtcgtcagctcgtgttgtgaaatgttgggttaagtcccgcaacgagcgcaacccttatcctttgttgccagcatgtaaagatgg
gaactcaaaggagactgccggtgacaaaccggaggaaggtggggatgacgtcaagtcatcatggcccttacgagtagggctaca
cacgtgctacaatggtgcatacagagggcggcgataccgcgaggtagagcgaatctcagaaagtgcatcgtagtccggattggagt
ctgcaactcgactccatgaagtcggaatcgctagtaatcgcaaatcagaatgttgcggtgaatacgttcccgggccttgtacacaccg
cccgtcacaccatgggagtgggttgtaccagaagtagatagcttaaccttcgggggggcgtttaccacggtatgattcatgactggg
gtgaagtcgtaacaaggtaaccgtaggggaacctgcgg
**SEQ ID NO: 2** (nucleotide sequence of 23 S rDNA of strain DD1)

agtaataacgaacgacacaggtataagaatacttgaggttgtatggttaagtgactaagcgtacaaggtggatgccttggcaatcaga
ggcgaagaaggacgtgctaatctgcgaaaagcttgggtgagttgataagaagcgtctaacccaagatatccgaatggggcaaccc
agtagatgaagaatctactatcaataaccgaatccataggttattgaggcaaaccgggagaactgaaacatctaagtaccccgagg
aaaagaaatcaaccgagattacgtcagtagcggcgagcgaaagcgtaagagccggcaagtgatagcatgaggattagaggaat
cggctgggaagccgggcggcacagggtgatagccccgtacttgaaaatcattgtgtggtactgagcttgcgagaagtagggcggga
cacgagaaatcctgtttgaagaagggggaccatcctccaaggctaaatactcctgattgaccgatagtgaaccagtactgtgaagg
aaaggcgaaaagaaccccggtgaggggagtgaaatagaacctgaaaccttgtacgtacaagcagtgggagcccgcgagggtga
ctgcgtaccttttgtataatgggtcagcgacttatattatgtagcgaggttaaccgaataggggagccgaagggaaaccgagtcttaact
gggcgtcgagttgcatgatatagacccgaaacccggtgatctagccatgggcaggttgaaggttgggtaacactaactggaggacc
gaaccgactaatgttgaaaaattagcggatgacctgtggctgggggtgaaaggccaatcaaaccgggagatagctggttctccccg
aaatctatttaggtagagccttatgtgaataccttcgggggtagagcactgtttcggctagggggccatcccggcttaccaacccgatgc
aaactgcgaataccgaagagtaatgcataggagacacacggcgggtgctaacgttcgtcgtggagagggaaacaacccagacc
gccagctaaggtcccaaagtttatattaagtgggaaacgaagtgggaaggcttagacagctaggatgttggcttagaagcagccatc
atttaaagaaagcgtaatagctcactagtcgagtcggcctgcgcggaagatgtaacggggctcaaatatagcaccgaagctgcggc
atcaggcgtaagcctgttgggtaggggagcgtcgtgtaagcggaagaaggtggttcgagagggctgctggacgtatcacgagtgcg
aatgctgacataagtaacgataaaacgggtgaaaaacccgttcgccggaagaccaagggttcctgtccaacgttaatcggggcag
ggtgagtcggcccctaaggcgaggctgaagagcgtagtcgatgggaaacgggttaatattcccgtacttgttataattgcgatgtggg
gacggagtaggttaggttatcgacctgttggaaaaggtcgtttaagttggtaggtggagcgtttaggcaaatccggacgcttatcaaca
ccgagagatgatgacgaggcgctaaggtgccgaagtaaccgataccacacttccaggaaaagccactaagcgtcagattataata
aaccgtactataaaccgacacaggtggtcaggtagagaatactcaggcgcttgagagaactcgggtgaaggaactaggcaaaata

gcaccgtaacttcgggagaaggtgcgccggcgtagattgtagaggtatacccttgaaggttgaaccggtcgaagtgacccgctggct
gcaactgtttattaaaaacacagcactctgcaaacacgaaagtggacgtatagggtgtgatgcctgcccggtgctggaaggttaattg
atggcgttatcgcaagagaagcgcctgatcgaagccccagtaaacggcggccgtaactataacggtcctaaggtagcgaaattcctt
gtcgggtaagttccgacctgcacgaatggcataatgatggccaggctgtctccacccgagactcagtgaaattgaaatcgccgtgaa
gatgcggtgtacccgcggctagacggaaagaccccgtgaacctttactatagcttgacactgaaccttgaattttgatgtgtaggatag
gtgggaggctttgaagcggtaacgccagttatcgtggagccatccttgaaataccaccctttaacgtttgatgttctaacgaagtgcccg
gaacgggtactcggacagtgtctggtgggtagtttgactggggcggtctcctcccaaagagtaacggaggagcacgaaggtttgcta
atgacggtcggacatcgtcaggttagtgcaatggtataagcaagcttaactgcgagacggacaagtcgagcaggtgcgaaagcag
gtcatagtgatccggtggttctgaatggaagggccatcgctcaacggataaaaggtactccggggataacaggctgataccgccca
agagttcatatcgacggcggtgtttggcacctcgatgtcggctcatcacatcctggggctgaagtaggtcccaagggtatggctgttcgc
catttaaagtggtacgcgagctgggtttaaaacgtcgtgagacagtttggtccctatctgccgtgggcgttggagaattgagaggggct
gctcctagtacgagaggaccggagtggacgcatcactggtgttccggttgtgtcgccagacgcattgccgggtagctacatgcggaa
gagataagtgctgaaagcatctaagcacgaaacttgcctcgagatgagttctcccagtatttaatactgtaagggttgttggagacgac
gacgtagataggccgggtgtgtaagcgttgcgagacgttgagctaaccggtactaattgcccgagaggcttagccatacaacgctca
agtgtttttggtagtgaaagttattacggaataagtaagtagtcagggaatcggct

SEQUENCE LISTING

<110>  BASF SE

<120>  Fermentative production of pyrazines using microorganisms of the
       genus Pasteurellaceae

<130>  B180921EP

<160>  2

<170>  PatentIn version 3.3

<210>  1
<211>  1517
<212>  DNA
<213>  Basfia succiniciproducens


<220>
<221>  MISC_FEATURE
<223>  16 S rDNA of strain DD1

<400>  1
tttgatcctg gctcagattg aacgctggcg gcaggcttaa cacatgcaag tcgaacggta      60

gcgggaggaa agcttgcttt ctttgccgac gagtggcgga cgggtgagta atgcttgggg     120

atctggctta tggaggggga taacgacggg aaactgtcgc taataccgcg taatatcttc     180

ggattaaagg gtgggacttt cgggccaccc gccataagat gagcccaagt gggattaggt     240

agttggtggg gtaaaggcct accaagccga cgatctctag ctggtctgag aggatgacca     300

gccacactgg aactgagaca cggtccagac tcctacggga ggcagcagtg gggaatattg     360

cacaatgggg ggaaccctga tgcagccatg ccgcgtgaat gaagaaggcc ttcgggttgt     420

aaagttcttt cggtgacgag gaaggtgttt gttttaatag gacaagcaat tgacgttaat     480

cacagaagaa gcaccggcta actccgtgcc agcagccgcg gtaatacgga gggtgcgagc     540

gttaatcgga ataactgggc gtaaagggca tgcaggcgga cttttaagtg agatgtgaaa     600

gccccgggct taacctggga attgcatttc agactgggag tctagagtac tttagggagg     660

ggtagaattc cacgtgtagc ggtgaaatgc gtagagatgt ggaggaatac cgaaggcgaa     720

ggcagcccct tgggaagata ctgacgctca tatgcgaaag cgtggggagc aaacaggatt     780

agataccctg gtagtccacg cggtaaacgc tgtcgatttg gggattgggc tttaggcctg     840

gtgctcgtag ctaacgtgat aaatcgaccg cctggggagt acggccgcaa ggttaaaact     900

caaatgaatt gacgggggcc cgcacaagcg gtggagcatg tggtttaatt cgatgcaacg     960

cgaagaacct tacctactct tgacatccag agaatcctgt agagatacgg gagtgccttc    1020

gggagctctg agacaggtgc tgcatggctg tcgtcagctc gtgttgtgaa atgttgggtt    1080

aagtcccgca acgagcgcaa cccttatcct ttgttgccag catgtaaaga tgggaactca    1140

aaggagactg ccggtgacaa accggaggaa ggtggggatg acgtcaagtc atcatggccc    1200

ttacgagtag ggctacacac gtgctacaat ggtgcataca gagggcggcg ataccgcgag 1260

gtagagcgaa tctcagaaag tgcatcgtag tccggattgg agtctgcaac tcgactccat 1320

gaagtcggaa tcgctagtaa tcgcaaatca gaatgttgcg gtgaatacgt tcccgggcct 1380

tgtacacacc gcccgtcaca ccatgggagt gggttgtacc agaagtagat agcttaacct 1440

tcgggggggg cgtttaccac ggtatgattc atgactgggg tgaagtcgta acaaggtaac 1500

cgtaggggaa cctgcgg 1517

<210> 2
<211> 3008
<212> DNA
<213> Basfia succiniciproducens

<220>
<221> MISC_FEATURE
<223> 23 S rDNA of strain DD1

<400> 2
agtaataacg aacgacacag gtataagaat acttgaggtt gtatggttaa gtgactaagc 60

gtacaaggtg gatgccttgg caatcagagg cgaagaagga cgtgctaatc tgcgaaaagc 120

ttgggtgagt tgataagaag cgtctaaccc aagatatccg aatggggcaa cccagtagat 180

gaagaatcta ctatcaataa ccgaatccat aggttattga ggcaaaccgg gagaactgaa 240

acatctaagt accccgagga aaagaaatca accgagatta cgtcagtagc ggcgagcgaa 300

agcgtaagag ccggcaagtg atagcatgag gattagagga atcggctggg aagccgggcg 360

gcacagggtg atagccccgt acttgaaaat cattgtgtgg tactgagctt gcgagaagta 420

gggcgggaca cgagaaatcc tgtttgaaga aggggggacc atcctccaag gctaaatact 480

cctgattgac cgatagtgaa ccagtactgt gaaggaaagg cgaaaagaac cccggtgagg 540

ggagtgaaat agaacctgaa accttgtacg tacaagcagt gggagcccgc gagggtgact 600

gcgtaccttt tgtataatgg gtcagcgact tatattatgt agcgaggtta accgaatagg 660

ggagccgaag ggaaaccgag tcttaactgg gcgtcgagtt gcatgatata gacccgaaac 720

ccggtgatct agccatgggc aggttgaagg ttgggtaaca ctaactggag gaccgaaccg 780

actaatgttg aaaaattagc ggatgacctg tggctggggg tgaaaggcca atcaaaccgg 840

gagatagctg gttctccccg aaatctattt aggtagagcc ttatgtgaat accttcgggg 900

gtagagcact gtttcggcta gggggccatc ccggcttacc aacccgatgc aaactgcgaa 960

taccgaagag taatgcatag gagacacacg gcgggtgcta acgttcgtcg tggagaggga 1020

aacaacccag accgccagct aaggtcccaa agtttatatt aagtgggaaa cgaagtggga 1080

aggcttagac agctaggatg ttggcttaga agcagccatc atttaaagaa agcgtaatag 1140

ctcactagtc gagtcggcct gcgcggaaga tgtaacgggg ctcaaatata gcaccgaagc 1200

tgcggcatca ggcgtaagcc tgttgggtag gggagcgtcg tgtaagcgga agaaggtggt 1260

tcgagagggc tgctggacgt atcacgagtg cgaatgctga cataagtaac gataaaacgg 1320

gtgaaaaacc cgttcgccgg aagaccaagg gttcctgtcc aacgttaatc ggggcagggt 1380

gagtcggccc ctaaggcgag gctgaagagc gtagtcgatg ggaaacgggt taatattccc 1440

gtacttgtta taattgcgat gtggggacgg agtaggttag gttatcgacc tgttggaaaa 1500

ggtcgtttaa gttggtaggt ggagcgttta ggcaaatccg gacgcttatc aacaccgaga 1560

gatgatgacg aggcgctaag gtgccgaagt aaccgatacc acacttccag gaaaagccac 1620

taagcgtcag attataataa accgtactat aaaccgacac aggtggtcag gtagagaata 1680

ctcaggcgct tgagagaact cgggtgaagg aactaggcaa aatagcaccg taacttcggg 1740

agaaggtgcg ccggcgtaga ttgtagaggt ataccettga aggttgaacc ggtcgaagtg 1800

acccgctggc tgcaactgtt tattaaaaac acagcactct gcaaacacga aagtggacgt 1860

atagggtgtg atgcctgccc ggtgctggaa ggttaattga tggcgttatc gcaagagaag 1920

cgcctgatcg aagccccagt aaacggcggc cgtaactata acggtcctaa ggtagcgaaa 1980

ttccttgtcg ggtaagttcc gacctgcacg aatggcataa tgatggccag gctgtctcca 2040

cccgagactc agtgaaattg aaatcgccgt gaagatgcgg tgtacccgcg gctagacgga 2100

aagaccccgt gaacctttac tatagcttga cactgaacct tgaattttga tgtgtaggat 2160

aggtgggagg ctttgaagcg gtaacgccag ttatcgtgga gccatccttg aaataccacc 2220

ctttaacgtt tgatgttcta acgaagtgcc cggaacgggt actcggacag tgtctggtgg 2280

gtagtttgac tggggcggtc tcctcccaaa gagtaacgga ggagcacgaa ggtttgctaa 2340

tgacggtcgg acatcgtcag gttagtgcaa tggtataagc aagcttaact gcgagacgga 2400

caagtcgagc aggtgcgaaa gcaggtcata gtgatccggt ggttctgaat ggaagggcca 2460

tcgctcaacg gataaaaggt actccgggga taacaggctg ataccgccca agagttcata 2520

tcgacggcgg tgtttggcac ctcgatgtcg gctcatcaca tcctggggct gaagtaggtc 2580

ccaagggtat ggctgttcgc catttaaagt ggtacgcgag ctgggtttaa aacgtcgtga 2640

gacagtttgg tccctatctg ccgtgggcgt tggagaattg agaggggctg ctcctagtac 2700

gagaggaccg gagtggacgc atcactggtg ttccggttgt gtcgccagac gcattgccgg 2760

gtagctacat gcggaagaga taagtgctga aagcatctaa gcacgaaact tgcctcgaga 2820

tgagttctcc cagtatttaa tactgtaagg gttgttggag acgacgacgt agataggccg 2880

ggtgtgtaag cgttgcgaga cgttgagcta accggtacta attgcccgag aggcttagcc 2940

atacaacgct caagtgtttt tggtagtgaa agttattacg gaataagtaa gtagtcaggg 3000

aatcggct 3008

## Claims

1. A process for producing pyrazines of the general formula (I) 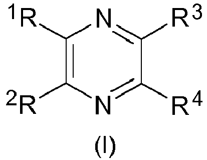 in which R¹, R², R³ and R⁴ independently from each other represent hydrogen, a C₁-C₁₀ alkyl group, a C₁-C₁₀ alkoxy group or a C₁-C₁₀ thioalkoxy group, wherein the process comprises the step of
i) cultivating microorganisms of the family *Pasteurellaceae* in a culture medium comprising at least one assimilable carbon source to allow the microorganisms to produce pyrazines of the general formula (I);
ii) recovering the pyrazines of the general formula (I) from the fermentation broth obtained in process step i).

2. The process according to claim 1, wherein the microorganism belongs to the genus *Basfia.*

3. The process according to claim 2, wherein the microorganism belongs to the species *Basfia succiniciproducens.*

4. The process according to claim 3, wherein the microorganism is *Basfia succiniciproducens* strain DD1 as deposited under DSM 18541 with the DSMZ, Germany.

5. The process according to anyone of claims 1 to 4, wherein the microorganism is a genetically modified microorganism in which at least one of the genes selected from the group consisting of *ldhA, pflA, pflD, wcaJ, pykA, fruA, ptsA, ptsH, udhA* and *rbsK* has been genetically modified.

6. The process according to anyone of claims 1 to 5, wherein the assimilable carbon source is selected from the group consisting of sucrose, maltose, D-fructose, D-glucose, D-xylose, L-arabinose, D-galactose, D-mannose, glycerol, decomposition products of starch, cellulose, hemicellulose and/or lignocellulose or mixtures thereof.

7. The process according to anyone of claims 1 to 6, wherein recovering the pyrazines of the general formula (I) from the fermentation broth in process step ii) comprises at least one treatment step selected from the group consisting of evaporation, filtration, sedimentation, crystallization, steam distillation, solvent extraction, dialysis, treatment with activated carbon, reverse osmosis, chromatography and a combination of at least two of these treatment steps.

8. The process according to claim 7, wherein recovering the pyrazines of the general formula (I) from the fermentation broth in process step ii) comprises the steps of
iia1) optionally evaporating the volatile components from the fermentation broth under reduced pressure at a temperature in the range from 50 to 90°C and collecting the evaporated components;
iib1) extracting the fermentation broth obtained in process step ii) or the evaporated components obtained in process step iia) with a solvent to obtained a solvent extract and evaporating the solvent from the solvent extract to obtain a residue comprising pyrazines of the general formula (I);
iic1) separating the pyrazines of the general formula (I) contained in said residue by means of chromatography.

9. The process according to claim 8, wherein the microorganisms are at least partially removed from the fermentation broth before process step iia1) is performed.

10. The process according to claim 7, wherein recovering the pyrazines of the general formula (I) from the fermentation broth in process step ii) comprises the steps of
iia2) separating the microorganisms from the fermentation broth;
iib2) treatment of the fermentation broth obtained in process step iia2) with activated carbon and separating the activated carbon from the fermentation broth;
iic2) treatment of the fermentation broth obtained in process step iib2) with reverse osmosis;
iid2) evaporation of at least a part of the water contained in the permeate obtained in process step iic2) to obtain a concentrated permeate;
iie2) crystallization of pyrazines of the general formula (I) from the concentrated permeate obtained in process step iid2).

11. The process according to anyone of claims 1 to 10, wherein the pyrazines of the general formula (I) are selected from the group consisting of 2,3,5-trimethyl pyrazine, 2,3,5-trimethyl-6-ethyl pyrazine, 2,3,5-trimethyl-6-propyl pyrazine, 2,3,5,6-tetramethyl pyrazine and mixtures of at least two thereof.

12. Use of microorganisms of the family *Pasteurellaceae* for the fermentative production of pyrazines of the general formula (I) 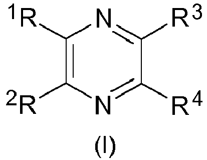 in which R¹, R², R³ and R⁴ independently from each other represent hydrogen, a C₁-C₁₀ alkyl group, a C₁-C₁₀ alkoxy group or a C₁-C₁₀ thioalkoxy group.

13. The use according to claim 12, wherein the microorganism belongs to the genus *Basfia.*

14. The use according to claim 13, wherein the microorganism belongs to the species *Basfia succiniciproducens.*

15. The use according to claim 14, wherein the microorganism is *Basfia succiniciproducens* strain DD1 as deposited under DSM 18541 with the DSMZ, Germany.
